# EUROPÄISCHE PATENTSCHRIFT

(11) **EP 1 818 022 B1**
(45) Veröffentlichungstag und Bekanntmachung des Hinweises auf die Patenterteilung: **04.04.2018**
(21) Anmeldenummer: 07101967.3
(22) Anmeldetag: 08.02.2007
(51) Int. Cl.: A61C 17/20, A61B 17/22

(54) **Vorrichtung und Verfahren zum Betrieb eines ultraschallgetriebenen Werkzeugs**
Device and method for operating an ultrasonic driven tool
Procédé et dispositif destinés au fonctionnement d'un outil à ultrasons

(30) Priorität: 13.02.2006 DE 102006006730
(43) Veröffentlichungstag der Anmeldung: 15.08.2007
(73) Patentinhaber: Sirona Dental Systems GmbH, 64625 Bensheim (DE)
(72) Erfinder: Rein, Matthias, 64653 Lorsch (DE)
(74) Vertreter: Sommer, Peter

(56) Entgegenhaltungen:
- US-A- 3 980 906
- US-A- 5 451 161
- US-A- 5 531 597
- US-A- 5 628 743

## Beschreibung

Die Erfindung betrifft ein Verfahren zum Betrieb eines Ultraschallsystems eines zahnärztlichen Instruments, wobei das Ultraschallsystem ein Bearbeitungswerkzeug und einen piezoelektrischen oder magnetostriktiven Antrieb umfasst und von dem Antrieb mit einer Resonanzfrequenz des Ultraschallsystems angeregt wird und wobei die über das Ultraschallsystem abgegebene Leistung veränderbar ist. Darüber hinaus betrifft die Erfindung ein zahnärztliches Instrument mit einem Ultraschallsystem zur Behandlung eines Patienten, wobei das Ultraschallsystem einen piezoelektrischen oder magnetostriktiven Antrieb und ein Bearbeitungswerkzeug umfasst und durch den Antrieb angeregt wird und wobei eine Antriebselektronik für den Antrieb vorgesehen ist.

### Stand der Technik

Im Stand der Technik sind zahnärztliche Handstücke mit Ultraschallantrieben bekannt, die mit Hilfe von piezoelektrischen Systemen angeregt werden. Die Anregung findet üblicherweise mit periodischen Rechteckimpulsen in der Grundresonanz statt, die neben einer Grundfrequenz zwangsläufig auch mehrere Oberwellen in vorbestimmter Frequenz und Amplitude enthalten. Abhängig von der Lage der Resonanzen des Ultraschallsystems werden somit zwangsläufig immer nur bestimmte Systemresonanzen angeregt, was zu einer vorbestimmten Schwingungsform des Ultraschallsystems führt. Bei diesen zahnärztlichen ultraschallbetriebenen Instrumenten kann eine Anpassung an die Erfordernisse der Behandlung lediglich durch eine Variation der Ausgangsleistung vorgenommen werden.

Aus der DE 36 41 058 A1 ist ein Zahnsteinentfernungsgerät bekannt, das eine Schaltungsanordnung zur Speisung eines Ultraschallgebers aufweist. Der Ultraschallgeber wird magnetostriktiv angeregt, wobei die Erregerspule durch Erregerimpulse angeregt wird. Die Schaltungsanordnung regelt die Impulsraten so, dass der magnetostriktive Wandler in Resonanz betrieben wird.

Aus der EP 0 411 473 B1 ist ein Ultraschallerzeuger für ein Gerät zur Zahnbehandlung mittels Ultraschall bekannt, welches einen piezoelektrischen Wandler aufweist, der mittels eines von einem Oszillator gespeisten Verstärker angetrieben wird, wobei ein Steuereinrichtung vorhanden ist, die eine Anpassung des Tastverhältnisses an den Resonanz-Arbeitspunkt vornimmt.

Aus der US 4,371,816 B1 ist ein Ultraschallscaler mit einer Kontrollschaltung bekannt, die die Ausgangsleistung und die Resonanzfrequenz des Scalers in Abhängigkeit des Lastzustandes des Scalers steuert.

In der US 5,531,597 A, auf deren Offenbarung die zweiteilige Form der Ansprüche 1 und 8 basiert, ist ein Verfahren zum Betrieb eines Ultraschallsystems eines zahnärztlichen Instruments beschrieben, wobei das Ultraschallsystem ein Bearbeitungswerkzeug und einen piezoelektrischen oder magnetostriktiven Antrieb umfasst, wobei das Bearbeitungswerkzeug mit einer Resonanzfrequenz schwingt. Die Grundfrequenz ist diejenige, deren Wellenlänge doppelt so lang ist wie das Werkzeug. Ferner wird erläutert, dass das Werkzeug auch mit harmonischen Frequenzen, d. h. Oberschwingungen der Grundfrequenz, schwingen kann, wobei als Beispiel die fünfte Oberschwingung genannt wird, bei welcher fünf Schwingungsknoten vorliegen.

In der US 5,628,743 A ist ein chirurgisches Instrument zum Schneiden von Gewebe offenbart. Das Instrument wird dabei mit einer ersten unteren Frequenz betrieben, was die Schneideigenschaften des Instruments verbessert. Zusätzlich kann das Instrument gleichzeitig mit einer zweiten höheren Frequenz angesteuert werden, womit die Koagulationseigenschaften beim Schneiden verbessert werden.

Ausgehend vom Stand der Technik besteht die erfindungsgemäße Aufgabe darin, ein dentales ultraschallbetriebenes Instrument anzugeben, das eine weitergehende Anpassung des Ultraschallantriebs an die Erfordernisse der Behandlung über die Ausgangsleistung und die Resonanzfrequenz hinaus ermöglicht.

### Darstellung der Erfindung

Diese Aufgabe wird gelöst durch ein Verfahren zum Betrieb eines Ultraschallsystems eines zahnärztlichen Instruments gemäß den kennzeichnenden Merkmalen des Anspruchs 1 sowie einem zahnärztlichen Instrument mit einem Ultraschallsystem gemäß den kennzeichnenden Merkmalen des Anspruchs 8.

Vorteilhafte Ausführungsformen der Erfindung gehen aus den abhängigen Patentansprüchen hervor.

Das Verfahren zum Betrieb eines Ultraschallsystems eines zahnärztlichen Instruments, wobei das Ultraschallsystem ein Bearbeitungswerkzeug und einen piezoelektrischen oder magnetostriktiven Antrieb umfasst und von dem Antrieb mit einer Resonanzfrequenz des Ultraschallsystems angeregt wird und wobei die über das Ultraschallsystem abgegebene Leistung veränderbar ist, sieht vor, daß der Antrieb des Ultraschallsystems mit wenigstens einer weiteren Resonanzfrequenz einer anderen Ordnung des Ultraschallsystems gezielt angeregt wird.

Bei einem derartigen Ultraschallsystem werden also gezielt wenigstens zwei Resonanzfrequenzen des Ultraschallsystems angeregt. Dies ermöglicht, definierte Schwingungszustände und damit definierte Bewegungsmuster der Spitze des Bearbeitungswerkzeugs einzustellen.

Das Ultraschallsystem wird in einem von mehreren aus der Art der Anregung resultierenden vorbestimmten Bewegungsmustern betrieben. Das Bewegungsmuster wird dabei durch mindestens zwei Betriebsparameter bestimmt. Diese Betriebsparameter können physikalische Größen der Schwingung betreffen oder auch anwenderfreundlicher ausgewählt sein, d.h., es können physikalisch komplexere Parameter gewählt werden, die für den Bediener intuitiver verständlich sind als physikalische Größen.

Solche anwenderfreundlichen Betriebsparameter zur Bestimmung des Bewegungsmusters des Ultraschallsystems sind eine Stärke und eine Aggressivität. Die Stärke entspricht dabei der von dem Ultaschallsystem abgegebenen Leistung und die Aggressivität des Ultraschallsystems sind zwei besonders praxistaugliche Betriebsparameter. Die Stärke wird durch die Amplitude bestimmt und die Aggressivität durch die Kraft, mit der sich die Spitze des Ultraschallsystems bewegt. Eine Spitze eines Ultraschallsystems bewegt sich vorwiegend stark, wenn sie eine hohe Amplitude mit wenig Kraft aufweist und vorwiegend aggressiv, wenn sie bei geringer Amplitude eine hohe Kraft aufweist. Auf diese Weise wird ermöglicht, ein nach diesem Verfahren betriebenes zahnärztliches Instrument beispielsweise zur Abtragung von dicken Ablagerungen mit hoher Abtragsleistung und trotzdem begrenzter Schwingungsamplitude zu betreiben. Andererseits wird es auch möglich, eine nur dünne Schicht zügig abzutragen.

Die Erfindung beruht darauf, dass durch Anregung eines komplexen Schwingungsmusters die Bewegungsform der Spitze des Bearbeitungswerkzeugs in drei Dimensionen veränderlich ist. So ist es beispielsweise möglich, neben bezüglich der Zahnoberfläche vorwiegend tangentialen Schwingungen der Spitze auch elliptische oder pulsierende Schwingungen zu erzeugen.

Vorteilhafterweise erfolgt die Anregung des Ultraschallsystems mit einem Frequenzgemisch, wobei das Frequenzgemisch eine Grundfrequenz aufweist, die einer Resonanzfrequenz des Ultraschallsystems entspricht. Ein derartiges Frequenzgemisch kann beispielsweise eine Rechteckschwingung oder eine Sägezahnschwingung sein. Rechteckschwingungen lassen sich in digitalen Systemen besonders einfach erzeugen.

Vorteilhafterweise wird eine Veränderung des zeitlichen Amplitudenverlaufs der Anregung des Antriebs vorgenommen, um eine Änderung der Art der Anregung des Ultraschallsystems zu bewirken. Bei analogen Systemen ist es beispielsweise möglich, Sinusschwingungen durch Verzerrer zu manipulieren, was zu einer Erzeugung eines Frequenzgemischs aus einer reinen Sinusschwingung führt.

Es erweist sich als besonders vorteilhaft, wenn das Ultraschallsystem vom Antrieb durch eine Überlagerung von mindestens zwei periodischen Rechtecksignalen mit unterschiedlicher Grundfrequenz angeregt wird. Eine Änderung der Art der Anregung des Ultraschallsystems erfolgt dann durch eine Änderung der Frequenz und/oder des Tastverhältnisses und/oder der Amplitude von mindestens einem der Rechtecksignale. Auf diese Weise ist es besonders einfach, eine gewünschte Schwingungsform des Ultraschallsystems einzustellen.

Vorteilhafterweise findet eine Anpassung der Anregung des Ultraschallsystems durch den Antrieb an die Lage der Resonanzfrequenzen des Ultraschallsystems statt. Die Lage der Resonanzfrequenzen des Ultraschallsystems ist abhängig von dessen mechanischem Aufbau, dessen Lastzustand und des Verschleißzustandes. Somit wird erreicht, dass das Ultraschallsystem immer optimal angeregt wird.

Vorteilhafterweise werden mehrere Resonanzfrequenzen des Ultraschallsystems mittels eines Anregungsimpulses ermittelt. Ein kurzer Anregungsimpuls enthält ein breites Spektrum an Frequenzen und ist somit in der Lage, das Ultraschallsystem breitbandig anzuregen. Die Resonanzfrequenzen können somit besonders leicht ermittelt werden.

Alternativ dazu ist es auch möglich, mehrere Resonanzfrequenzen des Ultraschallsystems mittels einer Strom-Spannungs- oder Leistungs-Zeit-Analyse zu ermitteln. Dabei wird ein Signal in das Ultraschallsystem eingespeist, das über das relevante Frequenzband durchgestimmt wird. Die Reaktion des Systems, beispielsweise der Verlauf des Stroms, wird dabei aufgenommen, da der Verlauf Rückschlüsse auf das Resonanzverhalten zulassen. Bei den Resonanzfrequenzen des Ultraschallsystems treten Extremwerte in der StromFrequenz-Kurve auf, die eine Identifikation der Resonanzfrequenzen des Ultraschallsystems erlauben. Hierzu kann eine übliche Wobbelschaltung vorgesehen sein, wie sie im Stand der Technik hinreichend bekannt ist.

Ein weiterer Gegenstand der Erfindung betrifft ein zahnärztliches Instrument mit einem Ultraschallsystem, wobei das Ultraschallsystem einen piezoelektrischen oder magnetostriktiven Antrieb und ein Bearbeitungswerkzeug umfasst und durch den Antrieb angeregt wird und wobei eine Antriebselektronik für den Antrieb vorgesehen ist, das eine Auswahleinrichtung zur Auswahl eines Betriebszustandes des Ultraschallsystems besitzt, wobei die Antriebselektronik den Antrieb in Abhängigkeit dieser Auswahl so steuert, dass der Antrieb gezielt eine Auswahl von mindestens zwei der Resonanzfrequenzen unterschiedlicher Ordnungen des Ultraschallsystems anregt.

Ein derartiges zahnärztliches Instrument ermöglicht dem Anwender, das Ultraschallsystem auf seine behandlungsspezifischen Bedürfnisse einzustellen. Die Bedienung eines derartigen zahnärztlichen Instruments ist besonders einfach, da die Antriebselektronik sämtliche über die Wahl des Betriebszustandes hinausgehenden Steuerfunktionen übernimmt.

Die Auswahleinrichtung so gestaltet, dass die unabhängige Auswahl von mindestens zwei Betriebsparametern des Ultraschallsystems möglich ist. Diese beiden Betriebsparameter sind die Stärke und die Aggressivität des Bewegungsmusters des Ultraschallsystems. Eine solche Auswahl erweist sich als besonders praxisgerecht.

Hier ist mit Vorteil eine Bedieneinrichtung mittels eines Hebels oder eines Schiebers, der in zwei Achsen unabhängig bewegt werden kann, vorzusehen.

### Kurzbeschreibung der Zeichnungen

Das erfindungsgemäße Verfahren wird anhand der Zeichnung erläutert. Es zeigen die:
- Fig. 1: ein zahnärztliches Handinstrument mit Ultraschallscaler, die
- Fig. 2: ein Prinzipbild der Antriebselektronik, die
- Fig. 3: einen Impedanzverlauf eines zahnärztlichen Ultraschallsystems, die
- Fig. 4A, B: Amplituden-Frequenz-Diagramme verschiedener Anregungsarten, die
- Fig. 5A, B: mögliche Bewegungsmuster der Spitze des Bearbeitungswerkzeugs.

### Ausführungsbeispiel

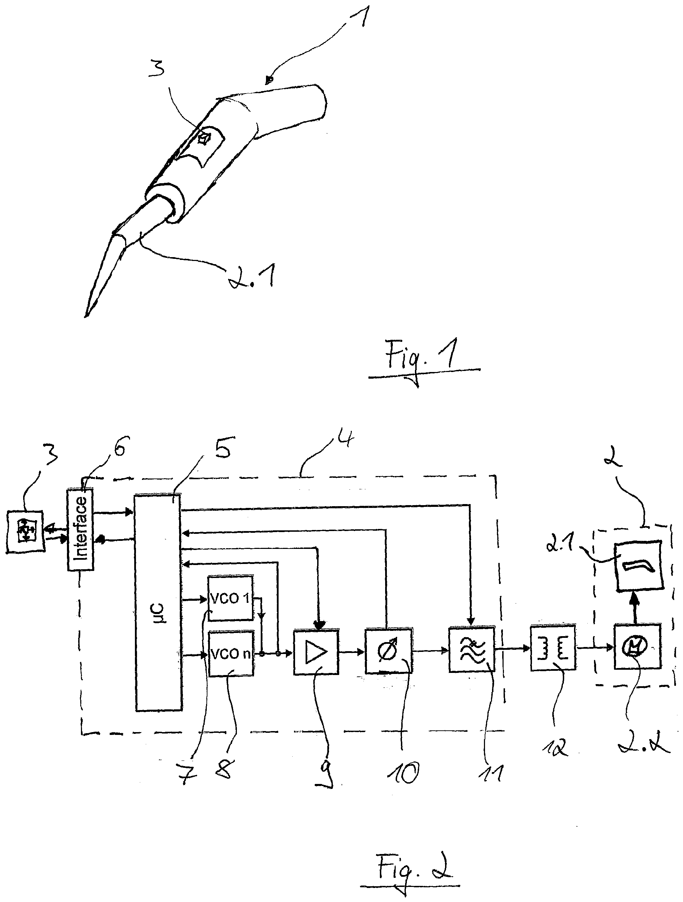

Die **Fig. 1** zeigt ein zahnärztliches Instrument 1 in Form eines Scalers mit einem Ultraschallsystem 2, umfassend ein Bearbeitungswerkzeug 2.1 und einen sich im Innern des Instruments 1 befindlichen, nicht dargestellten piezoelektrischen Antrieb für das Bearbeitungswerkzeug 2.1. Das Instrument 1 weist eine Auswahleinrichtung 3 auf, die eine unabhängige Einstellung des Betriebszustandes des Ultraschallsystems zumindest bezüglich zweier Parametern erlaubt. Der erste Parameter ist die Stärke der Bearbeitung, welche durch die Amplitude, mit der die Spitze des Bearbeitungswerkzeuges 2.1 schwingt, bestimmt ist und der zweite Parameter ist die Aggressivität, die durch die Kraft, mit der die Spitze des Bearbeitungswerkzeuges 2.1 schwingt, bestimmt ist.

Die **Fig. 2** zeigt die prinzipielle Funktionsweise der Antriebselektronik 4.

Das Kernelement der Antriebselektronik 4 ist ein Mikrocontroller 5, dem über ein Interface 6, welches mit der Auswahleinrichtung 3 verbunden ist, Vorgaben für den Betriebszustand des Ultraschallsystems 2 bereitgestellt werden. In Abhängigkeit dieser Vorgaben wählt der Mikrocontroller 5 ein passendes Betriebsprogramm und steuert zwei spannungsgesteuerten Oszillatoren 7, 8 durch die Vorgabe von jeweils einer Spannung an. Die spannungsgesteuerten Oszillatoren 7, 8 erzeugen eine Rechteckspannung mit einer Frequenz, die abhängig von der Spannungsvorgabe ist. Darüber hinaus können noch weitere nicht dargestellte spannungsgesteuerte Oszillatoren zur Erzeugung weiterer Frequenzen vorhanden sein.

Die von den Oszillatoren 7, 8 erzeugten Frequenzen werden dem Mikrocontroller 5 zur Regelung zurückgeführt, der bei einem Abweichen von dem gewünschten Betriebszustand eine Korrektur vornimmt.

Die von den beiden spannungsgesteuerten Oszillatoren 7, 8 erzeugten Frequenzen werden einem Verstärker 9 zugeführt, der diese auf die jeweilige Ausgangsleistung verstärkt, die dem Verstärker 9 vom Mikrocontroller 5 vorgegeben wird. Der Verstärker 9 ist so ausgebildet, dass er die Frequenzen in Abhängigkeit der Steuerungsvorgaben des Mikrocontrollers 5 selektiv verstärkt. Dazu kann der Verstärker 9 entweder mit mehreren Kanälen für die verschiedenen Frequenzen ausgestattet sein oder ein variables, frequenzabhängiges Verstärkungsprofil aufweisen. Im letzteren Fall können die Frequenzen der spannungsgesteuerten Oszillatoren 7, 8 bereits vor dem Verstärker 9 gemischt werden.

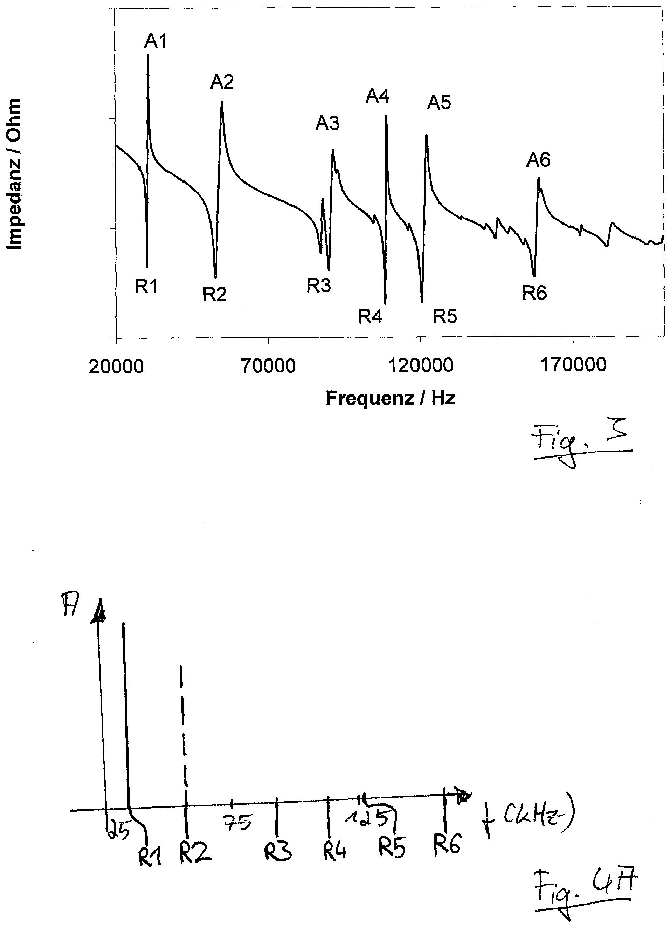

Eine Messvorrichtung 10 führt eine Strom- und Spannungsmessung an dem erzeugten und verstärkten Frequenzgemisch durch und führt diese Werte an den Mikrocontroller 5 zurück, der auf der Grundlage dieser Messwerte eine Korrektur der Steuerung des Antriebs 2.2 vornimmt. Da diese charakteristischen Größen auch von dem Schwingungszustand des Ultraschallsystems 2 abhängen, berücksichtigt diese Korrektur gleichzeitig den Last- und Verschleißzustand des Ultraschallsystems 2. Der Zusammenhang zwischen dem Schwingungszustand und der Impedanz des Schwingungssystems ist in Fig. 3 eingehender dargestellt.

Alternativ ist es denkbar, dass die Messvorrichtung 10 nur eine Strom- oder eine Spannungsmessung alleine an dem Frequenzgemisch durchführt oder eine Fast-FourierTransformation (FFT) des Frequenzgemisches vornimmt.

Der Mikrocontroller 5 steuert weiterhin ein Frequenzfilter 11, das unerwünschte Frequenzen selektiv oder bandweise aus dem von den spannungsgesteuerten Oszillatoren 7, 8 und dem Verstärker 9 erzeugten Frequenzgemisch herausfiltert.

Das von der Antriebselektronik 4 bereitgestellte Frequenzgemisch wird dann dem Antrieb 2.2 mittels eines Übertragers 12 übermittelt. Der Übertrager 12 ist als Transformator ausgebildet und transformiert im Falle eines piezoelektrischen Antriebs die Leistung auf eine höhere Ausgangsspannung.

Die Antriebselektronik 4 verfügt über eine spezielle Betriebsart zur Bestimmung der Lage der Resonanzfrequenzen R1-R6 des Ultraschallsystems 2, bei dem der spannungsgesteuerte Oszillator 7 vom Mikrocontroller 5 so angesteuert wird, dass er den betriebsrelevanten Frequenzgang des Ultraschallsystems 2 durchfährt, wobei die über das Messinstrument 10 erfassten Messwerte von dem Mikrocontroller 5 in Abhängigkeit von der Frequenz ausgewertet werden.

Die **Fig. 3** zeigt den Impedanzverlauf des Ultraschallsystems 2. Die Resonanzen des Ultraschallsystems 2 zeigen sich im Impedanzverlauf als Minima R1 bis R6. Zudem sind Antiresonanzpunkte A1 bis A6 dargestellt. Die Resonanzpunkte R1 bis R6 und die zugehörigen Antiresonanzpunkte A1 bis A6 sind ungleichmäßig über das Frequenzband verteilt. R1 liegt im Ausführungsbeispiel bei etwa 31 kHz, R2 bei etwa 57 kHz und R3 bei etwa 100 kHz.

In **Fig. 4A** wird die Überlagerung zweier Sinusschwingungen im Amplituden-Frequenz-Diagramm dargestellt. Auf der Frequenzachse ist weiterhin die Lage der Resonanzen R1-R6 des Ultraschallsystems 2 dargestellt. Die erste Sinusschwingung, dargestellt mit durchgezogener Linie, hat dabei eine Grundfrequenz von 31 kHz. Die zweite Sinusschwingung, dargestellt mit gestrichelter Linie, hat eine Grundfrequenz von 57 kHz. Wird das Ultraschallsystem 2, dessen Resonanzverlauf wie in Fig. 3 dargestellt aussieht, mit diesem Frequenzgemisch angeregt, so werden die Resonanzen R1 und R2 angesprochen.

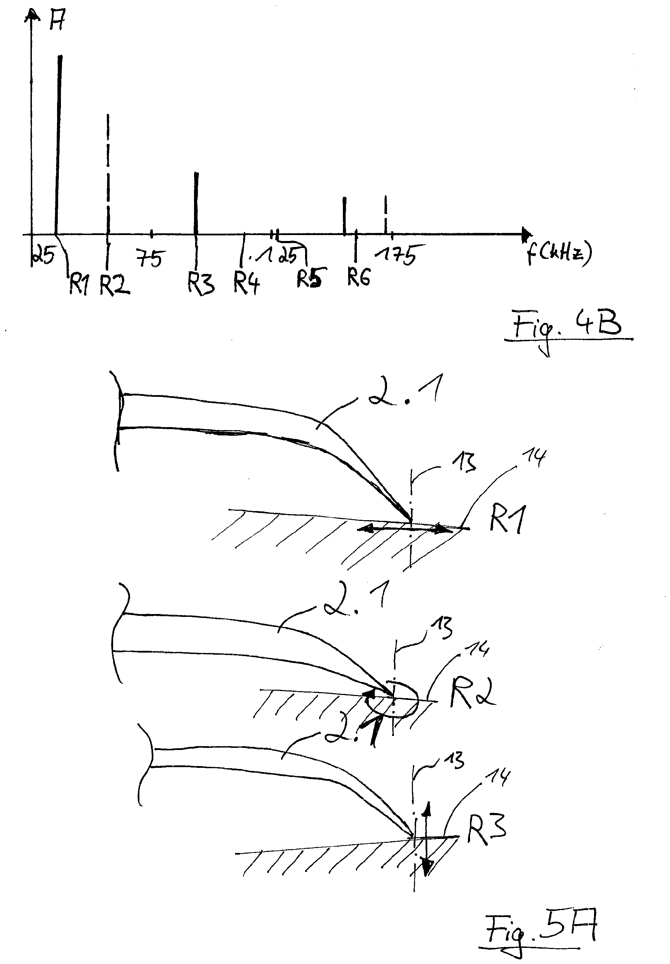

In **Fig. 4B** ist das Amplituden-Frequenz-Diagramm einer Überlagerung zweier Rechteckschwingungen zusammen mit der Lage der Resonanzfrequenzen R1-R6 dargestellt, wobei die erste Rechteckschwingung (durchgezogen dargestellt) eine Grundfrequenz von 31 kHz und die zweite Rechteckschwingung (gestrichelt dargestellt) eine Grundfrequenz von 57 kHz aufweist. Die ersten beiden weiteren Komponenten der ersten Rechteckschwingung bei 93 kHz und bei 155 kHz sind ebenfalls dargestellt. Der zweite Frequenzanteil der zweiten Rechteckschwingung liegt bei 171 kHz. Eine Anregung des Ultraschallsystems mit dieser Überlagerung zweier Rechteckschwingungen führt neben einer Anregung der Resonanzfrequenzen R1 und R2 auch noch zu Anregung der Resonanzfrequenz R6 durch den dritten Anteil der ersten Rechteckschwingung.

Um die Anregung von höheren Resonanzen wie beispielsweise R6 zu verhindern, kann das Frequenzfilter 11 als Tiefpassfilter ausgebildet werden, das Frequenzen oberhalb einer Grenzfrequenz herausfiltert.

In **Fig. 5A** sind die zu den Resonanzfrequenzen R1 bis R3 gehörigen Bewegungsformen der Spitze des Bearbeitungswerkzeuges 2.1 beispielhaft in vereinfachter Weise dargestellt, um die grundlegenden Prinzipien der Schwingungsformen und der Überlagerung mehrerer Schwingungen zu verdeutlichen.

Die Resonanzfrequenzen sowie die dazugehörigen Bewegungsformen der Spitze des Bearbeitungswerkzeuges 2.1 werden wesentlich von der Form des Bearbeitungswerkzeuges 2.1 bestimmt. Ist das Bearbeitungswerkzeug 2.1 dreidimensional asymmetrisch geformt, ist es möglich, dass eine Anregung in einer der Resonanzfrequenzen des Ultraschallsystems 2 Schwingungskomponenten in einer oder mehrerer der drei Raumrichtungen aufweist.

Beispielsweise regt R1 die Spitze des Bearbeitungswerkzeuges 2.1 des Ultraschallsystems 2 zu einer linearen Schwingung an, die im Wesentlichen senkrecht zur Oberflächennormalen 13 der Oberfläche des Zahnes 14 und entlang der Längsachse des Instruments 1 verläuft.

R2 kann beispielsweise zu einer im Wesentlichen elliptischen Schwingung in einer Ebene senkrecht zur Oberflächennormalen 13 führen.

R3 dagegen kann zu einer linearen Bewegung entlang der 0-berflächennormalen 13 führen.

Diese unterschiedlichen Bewegungsformen, die eine Anregung mit den reinen Resonanzfrequenzen R1 bis R3 an der Spitze des Bearbeitungswerkzeuges 2.1 verursacht, sind für sich genommen für die zahnärztliche Anwendung nicht besonders gut geeignet. Wünschenswert ist es, die Spitze 2.1 des Ultraschallsystems 2 so zu betreiben, dass ein Bewegungsmuster mit tangentialen Anteilen zur Oberfläche des Zahns 14 entsteht. Dies ist möglich durch die Überlagerung mindestens zweier Bewegungszustände durch die gleichzeitige Anregung von zwei oder mehr Resonanzfrequenzen des Ultraschallsystems 2, beispielsweise durch eine Überlagerung der Schwingungen R1 und R3.

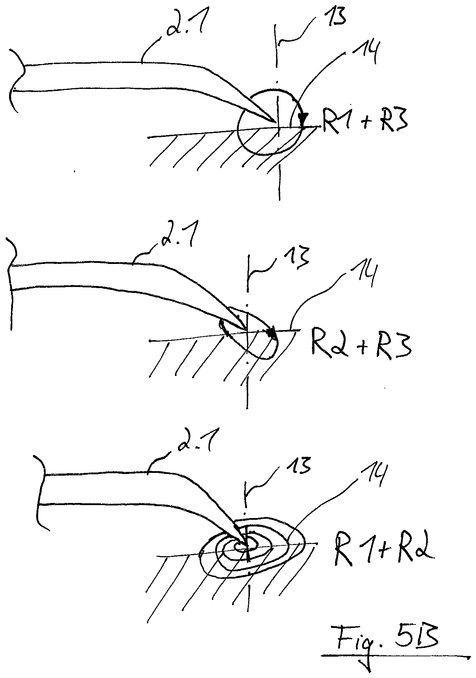

In **Fig. 5B** sind solche möglichen Überlagerungen auf der Grundlage der in Fig. 5A beispielhaft dargestellten Schwingungen dargestellt.

Eine Überlagerung von R1 und R3 führt zu einer elliptischen Oszillation der Spitze des Bearbeitungswerkzeuges 2.1 in einer Ebene, die im Wesentlichen senkrecht auf der Zahnoberfläche 14 steht.

Eine Überlagerung von R2 und R3 lässt die Spitze des Bearbeitungswerkzeuges elliptisch in einer Ebene schwingen, die geneigt zu der Oberflächennormalen 13 des Zahnes steht. Eine Überlagerung von R1 und R2 führt zu einem Bewegungsmuster, das in seinem Wirkungsbereich periodisch an- und abschwillt. Durch eine Veränderung der Amplituden der Anregungen ist es möglich, das Bewegungsmuster weiter zu beeinflussen und das Verhältnis der durch Überlagerung hervorgerufenen Halbachsen der Ellipsen einzustellen.

Darüber hinaus kann die Anregung mit zeitlich veränderlichen Amplituden vorgenommen werden, wodurch noch weitere Bewegungsmuster angeregt werden können. Beispielsweise ist es möglich, eine Resonanzfrequenz des Ultraschallsystems 2 pulsierend anzuregen.

### -Bezugszeichenliste

- 1: Instrument
- 2: Ultraschallschallsystem
- 2.1: Bearbeitungswerkzeug
- 2.2: Antrieb
- 3: Auswahleinrichtung
- 4: Antriebselektronik
- 5: Mikrocontroller
- 6: Interface
- 7: frequenzgesteuerter Oszillator
- 8: frequenzgesteuerter Oszillator
- 11: Frequenzfilter
- 12: Übertrager
- 13: Oberflächennormale
- 14: Zahn
- A1-A6: Antiresonanzfrequenz des Ultraschallsystems
- R1-R6: Resonanzfrequenz des Ultraschallsystems

## Patentansprüche

1. Verfahren zum Betrieb eines Ultraschallsystems (2) eines zahnärztlichen Instruments, wobei das Ultraschallsystem (2)ein Bearbeitungswerkzeug (2.1) und einen piezoelektrischen oder magnetostriktiven Antrieb (2.2) umfasst und von dem Antrieb (2.2) mit einer Resonanzfrequenz des Ultraschallsystems (2) angeregt wird und wobei die über das Ultraschallsystem (2) abgegebene Leistung veränderbar ist, **dadurch gekennzeichnet, dass** der Antrieb (2.2) mit wenigstens einer weiteren Resonanzfrequenz (R1-R6) einer anderen Ordnung des Ultraschallsystems (2) gezielt angeregt wird und das Ultraschallsystem (2) in einem von mehreren aus der Art der Anregung resultierenden vorbestimmten Bewegungsmustern betrieben wird, wobei das Bewegungsmuster durch mindestens zwei Betriebsparameter bestimmt wird und das Bewegungsmuster des Ultraschallsystems (2) einer vorgewählten Stärke und Aggressivität entsprechend eingestellt ist.

2. Verfahren nach Anspruch 1, **dadurch gekennzeichnet, dass** die Anregung des Ultraschallsystems (2) mit einem Frequenzgemisch erfolgt, wobei das Frequenzgemisch eine Grundfrequenz aufweist, die einer Resonanzfrequenz (R1-R6) des Ultraschallsystems (2) entspricht.

3. Verfahren nach Anspruch 1, **dadurch gekennzeichnet, dass** eine Veränderung des zeitlichen Amplitudenverlaufs der Anregung des Antriebs (2.2) vorgenommen wird, um eine Änderung der Art der Anregung des Ultraschallsystems (2) zu bewirken.

4. Verfahren nach Anspruch 1 oder 2, **dadurch gekennzeichnet, dass** das Ultraschallsystem (2) vom Antrieb (2.2) durch eine Überlagerung mindestens zweier periodischer Rechtecksignale mit unterschiedlicher Grundfrequenz (R1-R6) angeregt wird und eine Änderung der Art der Anregung des Ultraschallsystems (2) durch eine Änderung der Frequenz und/oder des Tastverhältnisses und/oder der Amplitude von mindestens einem der Rechtecksignale erfolgt.

5. Verfahren nach einem der Ansprüche 1 bis 4, **dadurch gekennzeichnet, dass** eine Anpassung der Anregung des Ultraschallsystems (2) durch den Antrieb (2.2) an die Abhängigkeit der Resonanzfrequenzen des Ultraschallsystems (2) von dessen Lastzustand und an den Verschleißzustand des Ultraschallsystems (2) vorgenommen wird.

6. Verfahren nach einem der Ansprüche 1 bis 5, **dadurch gekennzeichnet, dass** mehrere Resonanzfrequenzen (R1-R6) des Ultraschallsystems (2) mittels eines Anregungsimpulses ermittelt werden.

7. Verfahren nach einem der Ansprüche 1 bis 5, **dadurch gekennzeichnet, dass** mehrere Resonanzfrequenzen des Ultraschallsystems (2) mittels einer Strom-Spannungs- oder Leistungs-Zeit-Analyse ermittelt werden.

8. Zahnärztliches Instrument mit einem Ultraschallsystem (2) zur Behandlung eines Patienten, wobei das Ultraschallsystem (2) einen piezoelektrischen oder magnetostriktiven Antrieb (2.2) und ein Bearbeitungswerkzeug (2.1) umfasst und durch den Antrieb (2.2) angeregt wird und wobei eine Antriebselektronik (4) für den Antrieb (2.2) vorgesehen ist, **dadurch gekennzeichnet, dass** eine Auswahleinrichtung (3) zur Auswahl eines Betriebszustands des Ultraschallsystems (2) vorgesehen ist und dass die Antriebselektronik (4) den Antrieb (2.2) in Abhängigkeit dieser Auswahl so steuert, dass der Antrieb (2.2) gezielt eine Auswahl von mindestens zwei der Resonanzfrequenzen (R1-R6) unterschiedlicher Ordnungen des Ultraschallsystems (2) anregt und dass die Auswahleinrichtung (3) die unabhängige Auswahl von mindestens zwei Betriebsparametern des Ultraschallsystems (2), die Stärke und die Aggressivität, ermöglicht.

## Claims

1. Method for operating an ultrasound system (2) of a dental instrument, wherein the ultrasound system (2) comprises a processing tool (2.1) and a piezoelectric or magnetostrictive actuator (2.2) and said ultrasound system is excited by said actuator (2.2) with a resonant frequency of the ultrasound system (2), and wherein the output provided via the ultrasound system (2) is variable, **characterized in that** the actuator (2.2) is selectively excited with at least one further resonant frequency (R1-R6) of a different order of the ultrasound system (2) and the ultrasound system (2) is operated in one of a plurality of movement patterns resulting from and predetermined by the type of excitation, wherein the movement pattern is determined by means of at least two operating parameters and the movement pattern of the ultrasound system (2) is adjusted in accordance with a preselected strength and aggressiveness.

2. Method according to Claim 1, **characterized in that** the excitation of the ultrasound system (2) takes place with a frequency mixture, wherein the frequency mixture comprises a fundamental frequency that corresponds to a resonant frequency (R1-R6) of the ultrasound system (2).

3. Method according to Claim 1, **characterized in that** a modification of the temporal amplitude profile of the excitation of the actuator (2.2) is performed in order to effect a change in the type of excitation of the ultrasound system (2).

4. Method according to Claim 1 or 2, **characterized in that** the ultrasound system (2) is excited by the actuator (2.2) by means of a superposition of at least two periodic square wave signals with different fundamental frequencies (R1-R6) and a change in the type of excitation of the ultrasound system (2) is carried out by changing the frequency and/or the duty cycle and/or the amplitude of at least one of the square wave signals.

5. Method according to any of Claims 1 to 4, **characterized in that** the actuator (2.2) adjusts the excitation of the ultrasound system (2) to the dependence of the resonance frequencies of the ultrasound system (2) on its load state and to the wear condition of the ultrasound system (2).

6. Method according to any of Claims 1 to 5, **characterized in that** a plurality of resonance frequencies (R1-R6) of the ultrasound system (2) are determined by means of an excitation pulse.

7. Method according to any of Claims 1 to 5, **characterized in that** a plurality of resonant frequencies of the ultrasound system (2) are determined by means of a current-voltage or an output-time analysis.

8. Dental instrument with an ultrasound system (2) for treating a patient, wherein the ultrasound system (2) comprises a piezoelectric or magnetostrictive actuator (2.2) and a processing tool (2.1) and said ultrasound system is excited by said actuator (2.2), and wherein actuation electronics (4) for the actuator (2.2) are provided, **characterized in that** a selection means (3) for selecting an operating mode of the ultrasound system (2) is provided and that the actuation electronics (4) control the actuator (2.2) as a function of this selection in such a way that the actuator (2.2) selectively excites a selection of at least two of the resonance frequencies (R1-R6) of different orders of the ultrasound system (2), and that the selection means (3) allows the independent selection of at least two operating parameters of the ultrasound system (2), the strength and the aggressiveness.

## Revendications

1. Procédé d'exploitation d'un système à ultrasons (2) d'un instrument dentaire, le système à ultrasons (2) comprenant un outil de traitement (2.1) et un actionneur piézoélectrique ou magnétostrictif (2.2) et étant excité par l'actionneur (2.2) au moyen d'une fréquence de résonance du système à ultrasons (2), et la puissance fournie par le biais du système à ultrasons (2) étant modifiable, **caractérisé en ce que** l'actionneur (2.2) est excité de manière ciblée au moyen d'au moins une autre fréquence de résonance (R1-R6) d'un autre ordre du système à ultrasons (2), et le système à ultrasons (2) est exploité selon un modèle de mouvement parmi plusieurs modèles de mouvement prédéfinis résultant du type d'excitation, le modèle de mouvement étant déterminé par au moins deux paramètres d'exploitation, et le modèle de mouvement du système à ultrasons (2) étant réglé eu égard à une agressivité et une intensité choisies au préalable.

2. Procédé selon la revendication 1, **caractérisé en ce que** l'excitation du système à ultrasons (2) s'effectue au moyen d'un mélange de fréquences, le mélange de fréquences présentant une fréquence de base qui correspond à une fréquence de résonance (R1-R6) du système à ultrasons (2).

3. Procédé selon la revendication 1, **caractérisé en ce qu'**une modification de l'évolution dans le temps de l'amplitude de l'excitation de l'actionneur (2.2) est réalisée afin de produire une modification du type d'excitation du système à ultrasons (2).

4. Procédé selon la revendication 1 ou 2, **caractérisé en ce que** le système à ultrasons (2) est excité par l'actionneur (2.2) par le biais d'une superposition d'au moins deux signaux carrés périodiques de fréquences de base différentes (R1-R6), et une modification du type d'excitation du système à ultrasons (2) se produit suite à une modification de la fréquence et/ou du rapport cyclique et/ou de l'amplitude d'au moins un des signaux carrés.

5. Procédé selon l'une des revendications 1 à 4, **caractérisé en ce qu'**une adaptation de l'excitation du système à ultrasons (2) est réalisée par l'actionneur (2.2) en fonction de la dépendance des fréquences de résonance du système à ultrasons (2) par rapport à son état de charge et en fonction de l'état d'usure du système à ultrasons (2).

6. Procédé selon l'une des revendications 1 à 5, **caractérisé en ce que** plusieurs fréquences de résonance (R1-R6) du système à ultrasons (2) sont déterminées au moyen d'une impulsion d'excitation.

7. Procédé selon l'une des revendications 1 à 5, **caractérisé en ce que** plusieurs fréquences de résonance du système à ultrasons (2) sont déterminées au moyen d'une analyse de tension et d'intensité ou de puissance et de temps.

8. Instrument dentaire équipé d'un système à ultrasons (2) pour le traitement d'un patient, le système à ultrasons (2) comprenant un actionneur piézoélectrique ou magnétostrictif (2.2) et un outil de traitement (2.1) et étant excité par le biais de l'actionneur (2.2), un système électronique d'actionneur (4) étant prévu pour l'actionneur (2.2), **caractérisé en ce qu'**un dispositif de sélection (3) est prévu pour la sélection d'un état de fonctionnement du système à ultrasons (2) et **en ce que** le système électronique actionneur (4) de l'actionneur (2.2) est commandé en fonction de cette sélection, de sorte que l'actionneur (2.2) excite de manière ciblée une sélection d'au moins deux des fréquences de résonance (R1-R6) d'ordres différents du système à ultrasons (2) et que le dispositif de sélection (3) permet la sélection indépendante d'au moins deux paramètres de fonctionnement du système à ultrasons (2), à savoir l'intensité et l'agressivité.
